# EUROPEAN PATENT SPECIFICATION

(11) **EP 0 841 318 B1**
(45) Date of publication and mention of the grant of the patent: **27.11.2002**
(21) Application number: 97118997.2
(22) Date of filing: 31.10.1997
(51) Int. Cl.: C07C 33/12, C11B 9/00, A61K 7/46

(54) **Optical isomers of derivatives of campholenic aldehyde**
Optische Isomere von Derivaten von Kampholenaldehyd
Isomères optiques de dérivés de l'aldéhyde campholénique

(30) Priority: 06.11.1996 EP 96117716
(43) Date of publication of application: 13.05.1998
(73) Proprietor: Givaudan SA, 1214 Vernier-Genève (CH)
(72) Inventor: Bajgrowicz, Jerzy A., 8053 Zurich (CH); Frater, Georg, 8400 Winterthur (CH)
(74) Representative: Patentanwälte Schaad, Balass, Menzl & Partner AG

(56) References cited:
- EP-A- 0 203 528
- US-A- 4 052 341
- US-A- 4 173 585

## Description

The present invention relates to a mixture of the 4 diastereomers 1'S,2R,3S; 1'S,2S,3R; 1'S,2S,3S and 1'S,2R,3R of (E)-3-methyl-5-(2,2,3-trimethylcyclopent-3-en-1-yl)pent-4-en-2-ol, a mixture of the 4 diasteremomers 1'R,2S,3R; 1'R,2R,3S; 1'R,2R,3R and 1'R,2S,3S of (E)-3-methyl-5-(2,2,3-trimethylcyclopent-3-en-1-yl)pent-4-en-2-ol and to (1'S,2S,3R)-(E)-3-methyl-5-(2,2,3-trimethylcyclopent-3-en-1-yl)pent-4-en-2-ol, (1'R,2S,3R)-(E)-3-methyl-5-(2,2,3-trimethylcyclopent-3-en-1-yl)pent-4-en-2-ol and to a process for the manufacture of these compounds or mixtures. Further, the invention is related to any isomer mixture of (E)-3-methyl-5-(2,2,3-trimethylcyclopent-3-en-1-yl)pent-4-en-2-ol enriched in one or both of the compounds or mixtures, especially to an odorant composition containing any isomer mixture of (E)-3-methyl-5-(2,2,3-trimethylcyclopent-3-en-1-yl)pent-4-en-2-ol enriched in one or both of the above compounds or mixtures, and to the use of any one of the compounds or mixtures as odorants. Here and hereafter by the term "enriched" it is meant that an excess of one or both of the two compounds or mixtures mentioned at the beginning is present in an isomer mixture of (E)-3-methyl-5-(2,2,3-trimethylcyclopent-3-en-1-yl)pent-4-en-2-ol, i.e. a particular isomer or mixture of isomers is present in an over proportional amount. For example, a mixture of the 4 diastereomers of (1'S)-(E)-3-methyl-5-(2,2,3-trimethylcyclopent-3-en-1-yl)pent-4-en-2-ol is enriched in the 1'S,2S,3R isomer if it contains more than 25% of said 1'S,2S,3R isomer.

In spite of numerous studies in the domain of derivatives of campholenic aldehyde, the prediction of olfactory properties of different isomers and especially optical isomers remains a difficult challenge. Up to now it is not possible to predict which absolute configuration of carbon atoms of the five-membered ring and the side chain of such derivatives triggers the strongest, most substantive and/or natural sandalwood type odour.

East Indian sandalwood oil has been described as being "perhaps one of the most precious perfumery materials from antiquity down to modern times, and its popularity has shown no signs of waning" (E. Guenther in "The Essential Oils", D. Van Nostrand Company, Inc., New York, 1952, Vol. 5. p 173). This oil is widely used in perfumery and would be even more widely used except for its limited supply and high cost.

For many years a need existed for synthetic substitutes which could be used as sandalwood substitutes or as extenders.

The activity in the analysis of sandalwood oil and search for synthetic substitutes has been thoroughly reviewed (see E.-J. Brunke and E. Klein in "Fragrance Chemistry - The Science of the Sense of Smell", E.T. Theimer, Ed., Academic Press, New York, N.Y. 1982, p 397; K.H. Shankaranarayana and K. Parthasarathi, Perfumer and Flavorist, 9, 17, (1984); G. Ohloff, Ch. Fehr in "Perfumes - Art, Science and Technology", P.M. Mueller, D. Lamparsky, Eds., Elsevier Science Publishers, London, New York 1991, p 298; G. Frater and D. Lamparsky, ibid, p 561).

EP-A-203528 and US-Patent No. 4,696,766 are concerned inter alia with the compound (E)-3-methyl-5-(2,2,3-trimethylcyclopent-3-en-1-yl)pent-4-en-2-ol having the formula 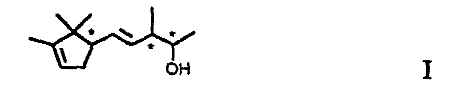 particularly with two pairs of enantiomers thereof. The compound has sandalwood olfactory properties. It was shown that the sandalwood olfactory properties of (E)-3-methyl-5-(2,2,3-trimethylcyclopent-3-en-1-yl)pent-4-en-2-ol depend on its relative configuration (see below). Because of the three asymmetric centers (*) in the compound of formula I eight optical isomers (four pairs of enantiomers) are expected. By gas-liquid chromatography on a Carbowax® 20M capillary column or by spinning band distillation the compound was separated in a lower boiling and a higher boiling component. Further gas-liquid chromatographic analysis on an ethylene glycol succinate capillary column resolved each of these two components confirming the presence of the four diastereomeric pairs of enantiomers.

So, the disclosure refers to two lower boiling pairs of enantiomers, i.e. 1'S*, 2R*,3S* and 1'S*.2S*,3R*, and to two higher boiling ones, i.e. 1'S*, 2R*,3R* and 1'S*,2S*,3S*. It was reported, that the two lower boiling pairs of enantiomers are the organoleptically active compounds possessing a very intense creamy, woody, musky, sandalwood odour and are valuable ingredients for use in fragrance compositions for its musky and sandalwood-like qualities. The mixture of these pairs of enantiomers is reported to be more intense than any of the campholenic aldehyde derivatives that have been previously reported. But nothing was disclosed either of the odour of the single enantiomers of the two most interesting pairs of enantiomers, i.e. 1'S*, 2R*,3S* and 1'S*,2S*,3R*, or of that of the other four stereoisomers which together constitutes a successful perfumery raw material sold under the name of Ebanol. It should be noted that the above mentioned lower boiling pairs of enantiomers are not commercially available.

The four pairs of enantiomers are shown below:

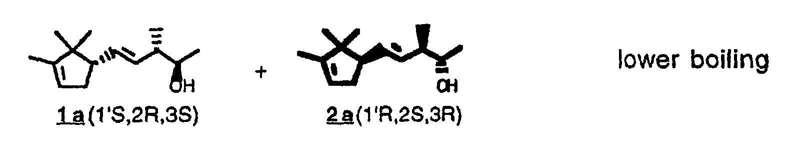

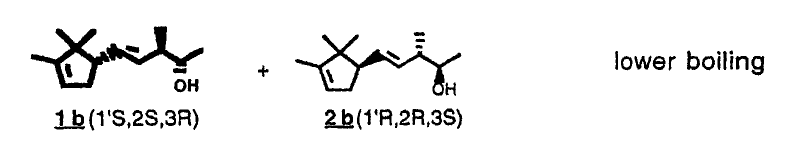

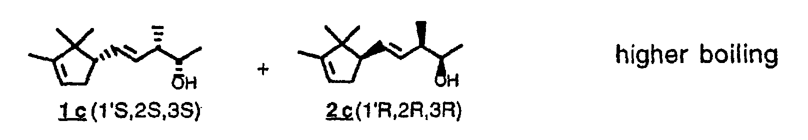

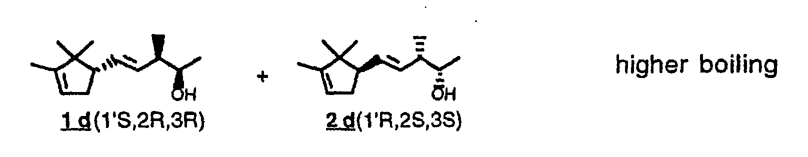

As described in EP-A-203528 the mixture of the diastereomers of compound I is prepared in a sequence of steps as outlined below.

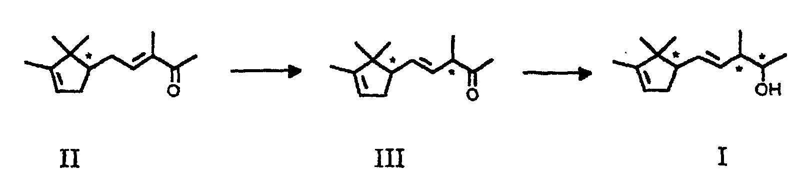

The compound II is an α,β-unsaturated ketone which is converted to the β,γ-unsaturated ketone III which then is converted to compound I by reducing the ketone carbonyl to the corresponding alcohol.

Compound II is prepared from campholenic aldehyde by an aldol condensation, whereby the campholenic aldeyde is prepared from α-pinene via α-pinene epoxide. This is a process well known in the art, see for example US-Patent No. 4,052,341.

It has now been found that the olfactory properties of each of the single optical isomers of this eight component mixture are crucial for the elaboration of a better performing (but still industrially viable) composition enriched in the more valuable isomer(s). This can improve significantly the quality of the commercialised raw material, offering more and better odour for the same weight, which is important not only for the economical but also environmental reasons.

In the synthesis of the compound I according to the above described reaction, the configuration of the campholenic aldehyde starting material depends on that of α-pinene which fixes the configuration of the C1' atom of the final product. The configuration of the two other asymmetric carbons of the compound, located in the side chain, namely C2 and C3, is more difficult to influence but can be discriminated either by rapidly developing asymmetric synthesis methods or by a relatively easy separation of the diastereomers. A systematic use of this recognition leads to the isolation of each single stereoisomer of the eight ones making compound I. So the organoleptic properties of each of the stereoisomers could be determined.

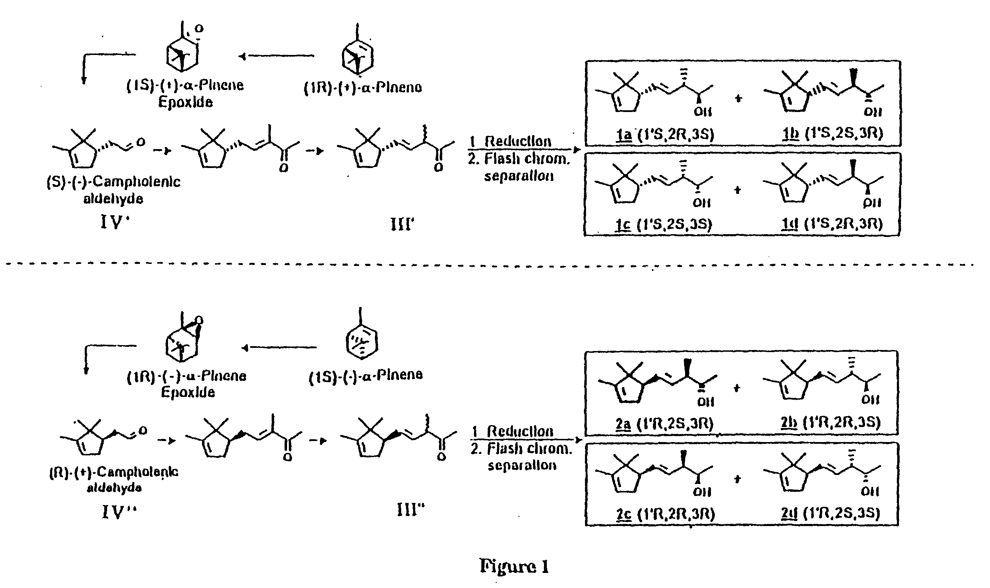

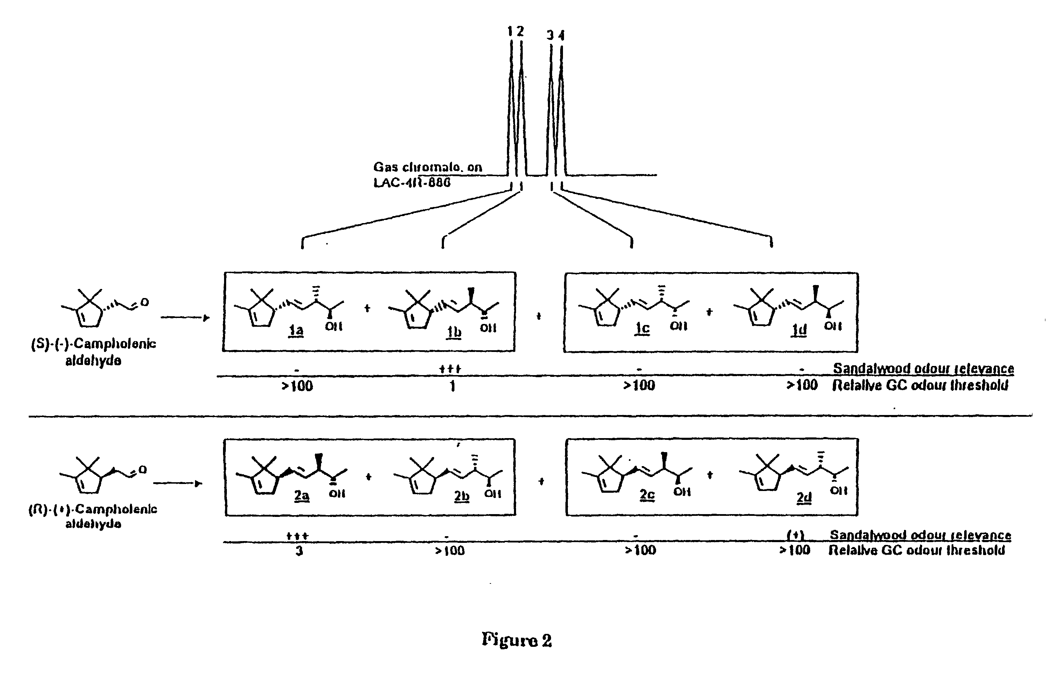

The foregoing is schematically shown in figures 1 and 2. If (1R)-(+)-α-pinene is used to produce campholenic aldehyde via pinene epoxide the product is (S)-(-)-campholenic aldehyde. Using this campholenic aldehyde as starting material for the production of compound II and then performing the reaction according to the above description (as shown in figure 1 in the upper part) the four diastereomers 1a, 1b, 1c and 1d of compound I are produced.

On the other hand, if (1S)-(-)-α-pinene is used to produce campholenic aldehyde via pinene epoxide the product is (1R)-(+)-campholenic aldehyde. Using this campholenic aldehyde as starting material for the production of compound II and then performing the reactions according to the above description (as also shown in figure 1) the other four stereoisomers, namely 2a, 2b, 2c, 2d, of compound I are produced. After the stereoisomers are separated by flash chromatography to yield 2 pairs of diastereomers these are further separated by gas chromatography as shown in figure 2. The final reduction of ketone III can be carried out in an asymmetric way (e. g. by using L-Selectride) yielding preferentially the two first eluted diastereomers, or diastereomeric pairs of enantiomers: 1a + 1b or 2a + 2b. Consequently, if in the starting material the enantiomeric excess is different from 100% two diastereomeric pairs of enantiomers 1a + 1b and 2a + 2b are preferentially obtained as it is demonstrated in Example 2 where the isomers (1'S*,2R*,3S*) and (1'S*,2S*,3R*) represent 79% of the product. In addition, figure 2 shows the sandalwood odours relevance as measured for each of the stereoisomers by determining its odour threshold.

The odour threshold is a physical property of the composition and is defined as being "its lowest concentration in air that can be consistently distinguished from pure air" (J.E. Amoore in "Fragrance Chemistry - The Science of the Sense of Smell", E.T. Theimer, Ed., Academic Press, New York, N.Y., 1982, p 34). The odour threshold value is expressed in terms of the weight of odorant per unit volume of air as determined by olfactometry, a technique known to those skilled in the art and discussed by Amoore. In essence, odour threshold value is the smallest amount of odorant that must be present in a unit volume of air to be detected, i.e. distinguished from the air itself.

Details of the procedure and the results are hereafter described and exemplified.

Four diastereomeric couples were synthesized and evaluated as such by the gas chromatography-sniff technique which allows to smell separately both of the two diastereomers of each couple. In this way the odour profiles and GC (gas chromatography) odour thresholds of all the eight optical isomers of (E)-3-methyl-5-(2,2,3-trimethylcyclopent-3-en-1-yl)pent-4-en-2-ol were obtained. The results are quantified in figure 2. Unexpectedly only one isomer out of the two enantiomers of both enantiomeric couples 1'S*,2R*,3S* and 1'S*,2S*,3R* turned out to be the vector of the strong natural sandalwood odour, namely 1'S,2S,3R and 1'R,2S,3R. These stereoisomers can each be obtained in pure form. The odour intensity ratio between these best stereoisomers (1'S,2S,3R)/ (1'R,2S,3R) is of -3-5:1. Their odours are by far the strongest and they are apparently the only ones of the eight constituents of the mixture that impart to it the natural, warm and lactonic sandalwood type odour note. This indicates that in this case the shape of the side chain as defined by the absolute configuration of the C2 and C3 carbons plays a key role in the perception of the sandalwood scent and is more important than that of the asymmetric carbon of the carbocycle.

The term 'in pure form' designates in the present context a material of being substantially free from other stereoisomers, preferably having at least 90% optical purity, more preferably having at least 95% optical purity.

Attribution of the absolute configuration of all stereoisomers cited in this paper is based on the GC retention time of diastereoisomeric pairs of enantiomers described in US-Patent No. 4,696,766 and the knowledge of the absolute configuration of the starting material. (S)-(-)-campholenic aldehyde and its (R)-(+)-enantiomer were synthesized according to the known procedure starting from (1R)-(+) and (1S)-(-)-α-pinene respectively. The pinenes with enantiomeric excess (e. e.) of >98% were provided by Aldrich. The campholenic aldehyde of Example 2 was obtained from an industrial quality of (1R)-(+)-α-pinene of e. e. = 43 %. The optical purity of the enantiomers of campholenic aldehyde was measured with the help of gas chromatography using an OV-1701/octakis(6-methyl-2,3-dipentyl)-γ-cyclodextrin chiral stationary phase and confirmed the e. e. of the pinenes. Gas chromatography analyses of the reaction products and intermediates were carried out on a Megabore DB 5 30 m x 0.53 mm column. Optical rotation measurements were performed on a Perkin-Elmer 241 polarimeter. The structure of the enantiomerically pure compounds and mixtures of diastereomers described in the examples were proven by their ¹H and ¹³C NMR, IR and mass spectra, identical with those of the corresponding enantiomeric mixtures described in above mentioned US-Patent No. 4,696,766. All synthesized products are colourless oils.

### Example 1.

### Preparation of (1'S)-(E)-3-methyl-5-(2,2,3-trimethylcyclopent-3-en-1-yl)pent-4-en-2-ol and separation of its stereoisomers

### a) Preparation of (1'S)-(E)-3-methyl-5-(2,2,3-trimethylcyclopent-3-en-1-yl)pent-3-en-2-one

This compound was obtained as the corresponding enantiomer mixture of Example II-1 of US-Patent No. 4,052,341 starting from 18.0 g of(S)-(-)-campholenic aldehyde and 52.1 g of butan-2-one and purified by distillation on a 5 cm Vigreux column. Yield: 15.1 g (62%). The product contained small amounts of other isomers but was used without further purification in the next step.

### b) Preparation of (1'S)-(E)-3-methyl-5-(2,2,3-trimethylcyclopent-3-en-1-yl)pent-4-en-2-one

7.0 g of the ketone from Example 1a) was added dropwise, during 30 min. to a stirred solution of 5.9 g of potassium *tert*-butoxide in 35 ml of dimethyl formamide (DMF) at 10°C. The yellow reaction mixture was stirred for 5 h at room temperature, then cooled to 0°C, treated rapidly with 25 ml of 20% aqueous acetic acid and extracted with 200 ml of methyl *tert*-butyl ether. The extract was washed with 2 x 50 ml of water, dried (MgSO₄) and concentrated *in vacuo* to give 5.2 g of crude product as slightly yellow oil. Purification by flash chromatography on silica gel using methyl *tert*-butyl ether/hexane 1:15 mixture as eluent gave 2.7 g (39 % yield) of (1'S)-(E)-3-methyl-5-(2,2,3-trimethylcyclopent-3-en-1-yl)pent-4-en-2-one. GC purity: 96 %; [α]_{D}²²= + 51.5° (c 1.03, ethanol).

### c) Preparation of (1'S)-(E)-3-methyl-5-(2,2,3-trimethylcyclopent-3-en-1-yl)pent-4-en-2-ol

A solution of 1.5 g of the β,γ-unsaturated ketone of Example 1b) in 10 ml of absolute ethanol was added dropwise at -5 °C and under nitrogen to 0.3 g of sodium borohydride suspended in 20 ml of the same solvent. Stirring was continued for 20 h at r. t. then the reaction mixture was poured on 150 ml of ice-cold 0.1 N hydrochloric acid and extracted with 2 x 100 ml of MTBE. The extract was washed with 2 x 100 ml of brine, dried (MgSO₄) and concentrated *in vacuo* to give 1.5 g of 93 % GC pure product (∼quantitative yield) as colourless oil.

### d) Separation of stereoisomers of (1'S)-(E)-3-Methyl-5-(2,2,3-trimethylcyclopent-3-en-1-yl)pent-4-en-2-ol

1.5 g of the crude product of the previous step was separated by flash chromatography on silica gel using methyl *tert*-butyl ether/hexane 1:5 mixture as eluent. Two fractions of only two constituents each were isolated:
fraction 1 (0.5 g; GC purity: >95 %) contains ∼3:2 mixture of isomers (1'S,2R,3S) and (1'S,2S,3R);
   [α]_{D}²² =+24.5° (c 1.01, ethanol); odour: sandalwood, dry, green, strong.
fraction 2 (0.25 g; GC purity: >95 %) contains ∼1:1 mixture of isomers (1'S,2S,3S) and (1'S,2R,3R);
   [α]_{D}²² =+27° (c 0.99, ethanol).; odour: celery, floral, fruity, green.

More of each isomer could have been isolated from the less pure fractions. Both pure fractions were separated by gas chromatography on the ethylene glycol succinate (LAC-4R-886) 39 m x 0.3 mm glass capillary column. Their constituents were evaluated by the GC-sniff technique. Odour quality and odour thresholds of single molecules are detailed in figure 2.

### Example 2.

### Preparation of (E)-3-methyl-5-(2,2,3-trimethylcyclopent-3-en-1-yl)-pent-4-en-2-ol containing ∼28% of isomer (1'S,2S,3R)

22 ml of 1 M solution of L-Selectride in tetrahydrofuran (THF) was added dropwise, under nitrogen, at 0 - 5 °C under stirring into 4.1 g of (E)-3-methyl-5-(2,2,3-trimethylcyclopent-3-en-1-yl)pent-4-en-2-one prepared according to Example 1b from an industrial quality of (S)-campholenic aldehyde (e.e. 43%) and dissolved in 40 ml of the same solvent. The reaction mixture was left to warm up to the room temperature and stirring continued for 4 h. 20 ml of methanol and 200 ml of brine was added successively and the aqueous phase was extracted with 3 x 200 ml of methyl tert-butyl ether (MTBE). The combined organic phases were dried (MgSO₄), the solvent evaporated *in vacuo* and the residue purified by flash chromatography (MTBE/hexane = 1/4) followed by bulb to bulb distillation at 0.1 Torr. The product (2.8 g; 67% yield) contained 40, 39, 4.5, and 6.5% of diastereomeric pairs of enantiomers (1'S*,2R*,3S*), (1'S*,2S*,3R*), (1'S*,2S*,3S*) and (1'S*,2R*,3R*) respectively, the enantiomeric ratio of each pair being 1'S/1'R = 71.5 : 28.5; [α]_{D}²²=+10.5° (c 1.00, ethanol).;
odour: woody, sandalwood, ambery with cedarwood and pine needle aspects, stronger and finer than Ebanol

### Example 3.

### Preparation of (1'R)-(E)-3-methyl-5-(2,2,3-trimethylcyclopent-3-en-1-yl)pent-4-en-2-ol and separation of the stereoisomers

The following (1'R) enantiomers were prepared starting from (R)-(+)-campholenic aldehyde in the same way as their (1'S)-analogues from Example 1:
a) (1'R)-(E)-3-Methyl-5-(2,2,3-trimethylcyclopent-3-en-1-yl)pent-3-en-2-one
b) (1'R)-(E)-3-methyl-5-(2,2,3-trimethylcyclopent-3-en-1-yl)pent-4-en-2-one GC purity: 98 %; [α]_{D}²²= -52.5° (c 1.06, ethanol).
c) (1'R)-(E)-3-methyl-5-(2,2,3-trimethylcyclopent-3-en-1-yl)pent-4-en-2-ol
d) Separation of stereoisomers of (1'R)-(E)-3-methyl-5-(2,2,3-trimethylcyclopent-3-en-1-yl)pent-4-en-2-ol

1.5 g (nearly quantitative yield) of the crude product of the previous step was separated by flash chromatography on silica gel using methyl *tert*-butyl ether/hexane 1:5 mixture as eluent. Two fractions of only two constituents each were isolated:
fraction 1 (0.5 g; GC purity: 91 %) contains ∼3:2 mixture of isomers (1'R,2S,3R) and (1'R,2R,3S);
   [α]_{D}²² =-25.5° (c 1.15, ethanol);
   odour: creamy, milky, sweet, sandalwood, weaker than that of the corresponding fraction 1 of Example 1d.
fraction 2 (0.32 g; GC purity: >95 %) contains ∼1:1 mixture of isomers (1'R,2R,3R) and (1'R,2S,3S);
   [α]_{D}²² =-24.5° (c 1.01, ethanol);
   odour: celery, woody.

More of each isomer could have been isolated from the less pure fractions. Both pure fractions were separated and evaluated. Odour quality and odour thresholds were determined by the GC-sniff technique as in Example 1d and presented in figure 2.

### Example 4.

This example shows the advantage of the isomers according to the invention in comparison to the state of the art product Ebanol which is a commercialised isomer mixture of (E)-3-methyl-5-(2,2,3-trimethylcyclopent-3-en-1-yl)pent-4-en-2-ol. A fine fragrance was produced containing the following ingredients.

| Ingredients | Quantity [g] |
|---|---|
| C-11 Aldehyde (undecylenic) 10% sol. in DPG | 0.3 |
| Allyl amyl glycolate | 0.4 |
| Benzyl acetate | 1.0 |
| Benzyl salicylate | 2.0 |
| Bergamot oil base | 8.0 |
| Cardamom oil (Ceylon) | 0.2 |
| Cassione (Firmenich), 10% sol. in DPG | 0.5 |
| Citronellyl acetate | 0.5 |
| Coumarin crist. | 0.5 |
| Cyclal C, 10% sol. in DPG | 0.5 |
| Dipropylene glycol | 10.6 |
| Ebanol | 1.0 |
| Galaxolide, 10% sol. in DEP | 20.0 |
| Geraniol | 0.8 |
| Hedione | 21.0 |
| cis-3-Hexenyl salicylate | 3.0 |
| Indolene, 10% sol. in DPG | 0.3 |
| Iso E super | 4.0 |
| cis-Jasmone, 10% sol. in DPG | 0.6 |
| Linalool synth. | 6.0 |
| Linalyl acetate | 4.0 |
| Mandarin oil (reconstitution) | 5.0 |
| Patchouli oil | 2.5 |
| Phenylethanol | 4.5 |
| Stemone | 0.3 |
| Vanillin | 2.5 |
| | 100.0 g |
| (DPG = dipropylene glycol; DEP = diethylphtalate) | |

When the quantity of Ebanol used in this composition was replaced by two times less (by weight) of the 3:2 mixture of (1'S,2R,3S) and (1'S,2S,3R)-(E)-3-methyl-5-(2,2,3-trimethylcyclopent-3-en-1-yl)pent-4-en-2-ol of Example 1d the sandalwood tonality of its odour was conserved and even strengthened. The sandalwood aspect was thus accentuated using less of the product.

### Example 5

A fragrance for a detergent powder or a softener was produced with the following ingredients and compared with the same fragrance but containing the isomers of fraction 1 of Example 1d instead of Ebanol.

| Ingredients | Quantity [g] |
|---|---|
| Agrumex | 5.0 |
| C-12 Aldehyde (MNA) | 0.03 |
| C-11 Aldehyde (iso) | 0.07 |
| Allyl cyclohexylpropionate | 2.0 |
| Anther | 1.0 |
| Bergamot oil base | 8.0 |
| 4-tert-Butylcyclohexyl acetate | 10.0 |
| Citronellol | 4.0 |
| Dihydromyrcenol | 3.0 |
| Dipropylene glycol | 3.0 |
| Dimethyl benzyl carbinyl acetate | 4.0 |
| Ebanol | 2.0 |
| Eucalyptus globulus oil (China) | 0.5 |
| Geranyl acetate | 1.5 |
| Fixolide | 7.0 |
| cis-Hexenol | 0.3 |
| α-Hexyl-cinnamaldehyde | 15.0 |
| β-Ionone | 3.0 |
| Iso E super | 2.0 |
| Isoraldeine 70 | 7.0 |
| cis-Jasmone | 0.1 |
| Lilial | 6.0 |
| Nonadyl | 1.0 |
| Rose oxide | 0.5 |
| Stemone | 0.5 |
| Undecavertol | 0.5 |
| Verdyl acetate | 8.0 |
| Verdyl propionate | 4.0 |
| Viridine | 1.0 |
| | 100.0 g |

Comparing the two fragrances the same effect as in Example 4 was observed on the humid and dried linen after washing or rinsing cycles.

The systematic chemical names of the trivial names of the individual components mentioned above are listed in standard works, e.g. Flavour and Fragrance Materials 1996, Allured Publishing Corporation, Carol Stream, Illinois, U.S.A. or Arctander, Perfume and Flavor Chemicals - 1969, published by the author, Montclair, New Jersey, U.S.A.

## Claims

1. An isomer mixture of(E)-3-methyl-5-(2,2,3-tru-nethylcyclopent-3-en-1-yl)pent-4-en-2-ol enriched in 1'S, 2R, 3S; 1'S. 2S, 3R; 1'S, 2S, 3S and 1'S, 2R, 3R diastereomers, or an isomer mixture of (E)-3-methyl-5-(2,2,3-trimethylcyclopent-3-en-1-yl)pent-4-en-2-ol enriched in 1'R, 2S, 3R,; 1'R, 2R, 3S; 1'R, 2R, 3R; and 1'R, 2S, 3S diastereomers.

2. A stereoisomer (1'S,2S,3R)-(E)-3-Methyl-5-(2,2,3-trimethylcyclopent-3-en-1-yl)pent-4-en-2-ol or (1'R,2S,3R)-(E)-3-Methyl-5-(2,2,3-trimethylcyclopent-3-en-1-yl)pent-4-en-2-ol.

3. A stereoisomer of claim 2 being at least 90% optically pure.

4. An isomer mixture of (E)-3-methyl-5-(2,2,3-trimethylcyclopent-3-en-1-yl)pent-4-en-2-ol enriched in one of the compounds of claim 2.

5. A process for preparing a mixture of claim 1 or of a compound of claim 2, **characterized in that** the mixture or the compound is synthetically prepared.

6. A process for preparing a mixture of claim 1 comprising using as appropriate, the starting material (1'S)-(E)-3-methyl-5-(2,2,3-trimethylcyclopent-3-en-1-yl)pent-4-en-2-one and reducing this to (1'S)-(E)-3-methyl-5-(2,2,3-trimethylcyclopent-3-en-1-yl)pent-4-en-2-ol; or the starting material (1'R)-(E)-3-methyl-5-(2,2,3-trimethylcyclopent-3-en-1-yl)pent-4-en-2-one and reducing this to (1'R)-(E)-3-methyl-5-(2,2,3-trimethylcyclopent-3-en-1-yl)pent-4-en-2-ol.

7. A process for preparing a stereoisomer of claim 2 or claim 3 comprising using as appropriate, the starting material (1'S)-(E)-3-methyl-5-(2,2,3-trimethylcyclopent-3-en-1-yl)pent-4-en-2-one and reducing this to (1'S)-(E)-3-methyl-5-(2,2,3-trimethylcyclopent-3-en-1-yl)pent-4-en-2-ol, which intermediate product is a mixture of the diastereomers 1'S,2R,3S; 1'S,2S,3R and 1'S,2S,3S; 1'S,2R,3R, separating the mixture of the diastereomers and isolating the isomer 1'S,2S,3R; or the starting material (1'R)-(E)-3-methyl-5-(2,2,3-trimethylcyclopent-3-en-1-yl)pent-4-en-2-one and reducing this to (1'R)-(E)-3-methyl-5-(2,2,3-trimethylcyclopent-3-en-1-yl)pent-4-en-2-ol, which intermediate product is a mixture of the diastereomers 1'R,2S,3R; 1'R,2R,3S and 1'R,2R,3R; 1'R,2S,3S, separating the mixture of the diastereomers and isolating the isomer 1'R,2S,3R.

8. The process of claim 6 or 7 **characterized in that** the starting material (1'S)-(E)-3-methyl-5-(2,2,3-trimethylcyclopent-3-en-1-yl)pent-4-en-2-one is synthesized from (S)-(-)-campholenic aldehyde in a manner known per se; and the starting material (1'R)-(E)-3-methyl-5-(2,2,3-trimethylcyclopent-3-en-1-yl)pent-4-en-2-one is synthesized from (R)-(+)-campholenic aldehyde in a manner known per se.

9. The use of the mixture according to claim 1 or claim 4 or of the stereoisomer according to claim 2 or 3 as an odorant.

10. An odorant composition containing any isomer mixture of (E)-3-methyl-5-(2,2,3-trimethylcyclopent-3-en-1-yl)pent-4-en-2-ol enriched in at least one of the mixtures of claims 1 or 4 and/or at least one of the stereoisomer of claim 2 or 3.

11. The process according to any one of the claims 6, 7 and 8 **characterized in that** the starting material is present in an enantiomeric excess.

12. The process according to claim 11, **characterized in that** the reduction of the starting material is carried out in an asymmetric way.

## Patentansprüche

1. Eine Isomerenmischung von (E)-3-Methyl-5-(2,2,3,trimethylcyclopent-3-en-1-yl)pent-4-en-2-ol angereichert mit 1'S,2R,3S; 1'S,2S,3R; 1'S,2S,3S und 1'S,2R,3R Diastereomeren oder eine Isomerenmischung von (E)-3-Methyl-5-(2,2,3-trimethylcyclopent-3-en-1-yl)pent-4-en-2-ol angereichert mit 1'R,2S,3R-1'R,2R,3S; 1'R,2R,3R; und 1'R,2S,3S Diastereomeren.

2. Ein Stereoisomer (1'S,2S,3R)-(E)-3-Methyl-5-(2,2,3-trimethylcyclopent-3-en-1-yl)pent-4-en-2-ol oder (1'R,2S,3R)-(E)-3-Methyl-5-(2,2,3-trimethylcyclopent-3-en-1-yl)pent-4-en-2-ol.

3. Ein Stereoisomer nach Anspruch 2, das mindestens 90% optisch rein ist.

4. Eine Isomerenmischung von (E)-3-Methyl-5-(2,2,3-trimethylcyclopent-3-en-1-yl)pent-4-en-2-ol angereichert mit einer der Verbindungen nach Anspruch 2.

5. Ein Verfahren zur Herstellung einer Mischung nach Anspruch 1 oder einer Verbindung nach Anspruch 2 **dadurch gekennzeichnet, dass** die Mischung oder die Verbindung synthetisch hergestellt ist.

6. Ein Verfahren zur Herstellung einer Mischung nach Anspruch 1 enthaltend die Verwendung von (1'S)-(E)-3-Methyl-5-(2,2,3-trimethylcyclopent-3-en-1-yl)-pent-4-en-2-on als geeignetes Ausgangsmaterial und Reduzieren desselben zu (1'S)-(E)-3-Methyl-5-(2,2,3-trimethylcyclopent-3-en-1-yl)pent-4-en-2-ol; oder von (l'R)-(E)-3-methyl-5-(2,2,3-trimethylcyclopent-3-en-1-yl)pent-4-en-2-on als geeignetes Ausgangsmaterial und reduzieren desselben zu (l'R)-(E)-3-Methyl-5-(2,2,3-trimethylcyclopent-3-en-1-yl)pent-4-en-2-ol.

7. Ein Verfahren zur Herstellung eines Stereoisomers nach Anspruch 2 oder Anspruch 3, enthaltend die Verwendung von (1'S)-(E)-3-Methyl-5-(2,2,3-trimethylcyclopent-3-en-1-yl)pent-4-en-2-on als geeignetes Ausgangsmaterial und Reduzieren desselben zu (1'S)-(E)-3-Methyl-5-(2,2,3-trimethylcyclopent-3-en-1-yl)pent-4-en-2-ol, wobei das Zwischenprodukt eine Mischung der Diastereomeren 1'S,2R,3S; 1'S,2S,3R und 1'S,2S,3S; 1'S,2R,3R ist, die Mischung der Diastereomeren getrennt wird und das Isomer 1'S,2S,3 isoliert wird; oder von (1'R)-(E)-3-Methyl-5-(2,2,3-trimethylcyclopent-3-en-1-yl)pent-4-en-2-on als geeignetes Ausgangsmaterial und Reduzieren desselben zu (1'R)-(E)-3-Methyl-5-(2,2,3-trimethylcyclopent-3-en-1-yl)pent-4-en-2-ol, wobei das Zwischenprodukt eine Mischung der Diastereomeren 1'R,2S,3R;1'R,2R,3S und 1'R,2R,3R; 1'R,2S,3S ist, die Mischung der Diastereomeren getrennt wird und das Isomer 1'R,2S,3R isoliert wird.

8. Das Verfahren nach Anspruch 6 oder 7 **dadurch gekennzeichnet, dass** das Ausgangsmaterial (1'S)-(E)-3-Methyl-5-(2,2,3-trimethylcyclopent-3-en-1-yl)pent-4-en-2-on von (S)-(-)-campholenischem Aldehyd nach einer bekannten Methode synthetisiert wird; und das Ausgangsmaterial (1'R)-(E)-3-Methyl-5-(2,2,3-trimethylcyclopent-3-en-1-yl)pent-4-en-2-on von (R)-(+)-campholenischem Aldehyd nach einer bekannten Methode synthetisiert wird.

9. Die Verwendung der Mischung nach Anspruch 1 oder 4 oder des Stereoisomers nach Anspruch 2 oder 3 als ein Duftstoff.

10. Eine duftende Zusammensetzung enthaltend irgendeine Isomerenmischung von (E)-3-Methyl-5-(2,2,3-trimethylcyclopent-3-en-1-yl)pent-4-en-2-ol angereichert mit mindestens einer der Mischungen nach Anspruch 1 oder 4 und/oder mindestens eines der Stereoisomeren von Anspruch 2 oder 3.

11. Verfahren nach einem der Ansprüche 6,7 und 8, **dadurch gekennzeichnet, dass** das Ausgangsmaterial in einem enantiomeren Überschuss anwesend ist.

12. Das Verfahren nach Anspruch 11, **dadurch gekennzeichnet, dass** die Reduktion des Ausgangsmaterials auf eine asymmetrische Weise ausgeführt wird.

## Revendications

1. Mélange d'isomères de (E)-3-méthyl-5-(2,2,3-triméthylcyclopent-3-én-1-yl)pent-4-én-2-ol enrichi en diastéréoisomères 1'S,2R,3S ; 1'S,2S,3R ; 1'S,2S,3S et 1'S,2R,3R ou mélange d'isomères de (E)-3-méthyl-5-(2,2,3-triméthylcyclopent-3-én-1-yl)pent-4-én-2-ol enrichi en diastéréoisomères 1'R,2S,3R ; 1'R,2R,3S ; 1'R,2R,3R et 1'R,2S,3S.

2. Stéréoisomère (1'S,23,3R)-(E)-3-méthyl-5-(2,2,3-triméthylcyclopent-3-én-1-yl)pent-4-én-2-ol ou (1'R,2S,3R) (E)-3-méthyl-5-(2,2,3-triméthylcyclopent-3-én-1-yl)pent-4-én-2-ol.

3. Stéréoisomère selon la revendication 2 étant au moins optiquement pur à 90%.

4. Mélange d'isomères de (E)-3-méthyl-5-(2,2,3-tri-méthylcyclopent-3-én-1-yl)pent-4-én-2-ol enrichi en un des composés selon la revendication 2.

5. Procédé pour préparer un mélange selon la revendication 1 ou composé selon la revendication 2, **caractérisé en ce que** le mélange ou le composé est préparé de façon synthétique.

6. Procédé pour préparer un mélange selon la revendication 1, comprenant l'utilisation de façon appropriée de la matière de départ (1'S)-(E)-3-méthyl-5-(2,2,3-triméthylcyclo-pent-3-én-1-yl)pent-4-én-2-one et la réduction de celle-ci en (1'S)-(E)-3-méthyl-5-(2,2,3-triméthylcyclopent-3-én-1-yl)pent-4-én-2-ol; ou de la matière de départ (1'R)-(E)-3-méthyl-5-(2,2,3-triméthylcyclopent-3-én-1-yl)pent-4-én-2-one et la réduction de celle-ci en (1'R)-(E)-3-méthyl-5-(2,2,3-triméthylcyclopent-3-én-1-yl)pent-4-én-2-ol.

7. Procédé pour préparer un stéréoisomère selon la revendication 2 ou 3, comprenant l'utilisation de façon appropriée de la matière de départ (1'S)-(E)-3-méthyl-5-(2,2,3-triméthylcyclopent-3-én-1-yl)pent-4-én-2-one et la réduction de celle-ci en (1'S)-(E)-3-méthyl-5-(2,2,3-triméthylcyclopent-3-én-1-yl)pent-4-én-2-ol, lequel produit intermédiaire est un mélange des diastéréoisomères 1'S,2R,3S ; 1'S,2S,3R et 1'S,2S,3S ; 1'S,2R,3R,1a séparation du mélange des diastéréoisomères et l'isolation de l'isomère 1'S,2S,3R ; ou de la matière de départ (1'R)-(E)-3-méthyl-5-(2,2,3-triméthylcyclopent-3-én-1-yl)pent-4-én-2-one et la réduction de celle-ci en (1'R)-(E)-3-méthyl-5-(2,2,3-triméthylcyclopent-3-én-1-yl)pent-4-én-2-ol,lequel produit intermédiaire est un mélange des diastéréoisomères 1'R,2S,3R ; 1'R,2R,3S et 1'R,2R,3R ; 1'R,2S,3S,1a séparation du mélange des diastéréoisomères et l'isolation de l'isomère 1'R,2S,3R.

8. Procédé selon la revendication 6 ou 7, **caractérisé en ce que** la matière de départ (1'S)-(E)-3-méthyl-5-(2,2,3-triméthylcyclopent-3-én-1-yl)pent-4-én-2-one est synthétisée à partir de l'aldéhyde (S)-(-)-campholénique d'une manière connue en elle-même ; et la matière de départ (1'R)-(E)-3-méthyl-5-(2,2,3-triméthylcyclopent-3-én-1-yl)pent-4-én-2-one est synthétisée à partir de l'aldéhyde (R)-(+)-campholénique d'une manière connue en elle-même.

9. Utilisation du mélange selon la revendication 1 ou 4 ou des stéréoisomères selon la revendication 2 ou 3 en tant que substance odorante.

10. Composition odorante contenant tout mélange d'isomères de (E)-3-méthyl-5-(2,2,3-triméthylcyclopent-3-én-1-yl)pent-4-én-2-ol enrichi en au moins un des mélanges selon la revendication 1 ou 4 et/ou au moins un des stéréoisomères selon la revendication 2 ou 3.

11. Procédé selon l'une quelconque des revendications 6, 7 et 8, **caractérisé en ce que** la matière de départ est présente en excès énantiomère.

12. Procédé selon la revendication 11, **caractérisé en ce que** la réduction de la matière de départ est effectuée de façon asymétrique.
